(19) Europäisches Patentamt
European Patent Office
Office européen des brevets

(11) **EP 4 061 310 B1**

(12) **EUROPÄISCHE PATENTSCHRIFT**

(45) Veröffentlichungstag und Bekanntmachung des Hinweises auf die Patenterteilung:
**20.05.2026 Patentblatt 2026/21**

(21) Anmeldenummer: **20807771.9**

(22) Anmeldetag: **20.11.2020**

(51) Internationale Patentklassifikation (IPC):
*A61K 31/192* *(2006.01)*    *A61K 31/19* *(2006.01)*
*A61P 17/10* *(2006.01)*    *A61K 8/365* *(2006.01)*
*A61K 8/368* *(2006.01)*    *A61Q 19/00* *(2006.01)*

(52) Gemeinsame Patentklassifikation (CPC):
(C-Sets verfügbar)
**A61P 17/10; A61K 8/365; A61K 8/368;**
**A61K 31/19; A61K 31/192; A61Q 19/00**    (Forts.)

(86) Internationale Anmeldenummer:
**PCT/EP2020/082863**

(87) Internationale Veröffentlichungsnummer:
**WO 2021/099555 (27.05.2021 Gazette 2021/21)**

(54) **KOSMETISCHE ZUBEREITUNG MIT ANISSÄURE UND LÄVULINSÄURE MIT SELEKTIVER ANTIMIKROBIELLER WIRKSAMKEIT**

COSMETIC PREPARATION COMPRISING ANISIC ACID AND LEVULINIC ACID WITH SELECTIVE ANTIMICROBIAL ACTIVITY

PRÉPARATION COSMÉTIQUE COMPRENANT L'ACIDE ANISIQUE ET L'ACIDE LÉVULONIQUE À ACTIVITÉ ANTIMICROBIENNE SÉLECTIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: 20.11.2019 DE 102019217898
04.09.2020 EP 20194680

(43) Veröffentlichungstag der Anmeldung:
**28.09.2022 Patentblatt 2022/39**

(73) Patentinhaber: **Beiersdorf AG**
**22529 Hamburg (DE)**

(72) Erfinder:
• **TRAUPE, Bernd**
**24568 Hamburg (DE)**
• **BIERGIESSER, Helga**
**21465 Reinbek (DE)**
• **FIRYN, Andreas**
**21493 Schwarzenbek (DE)**
• **GÖDDERTZ, Dominik**
**22457 Hamburg (DE)**
• **TREDE, Lucia Zanforlin**
**20255 Hamburg (DE)**
• **AVILA, Luis Arturo Carbajal**
**20253 Hamburg (DE)**
• **KÖRBL, Birthe**
**20535 Hamburg (DE)**
• **FÖLSTER, Heike**
**22149 Hamburg (DE)**
• **HAMANN, Tina**
**24864 Brodersby-Goltoft (DE)**
• **SCHÖNDIENST, Petra**
**25436 Tornesch (DE)**
• **RICHTER, Daniel**
**22850 Norderstedt (DE)**

(74) Vertreter: **Eisenführ Speiser**
**Patentanwälte Rechtsanwälte PartGmbB**
**Johannes-Brahms-Platz 1**
**20355 Hamburg (DE)**

(56) Entgegenhaltungen:
CN-A- 109 157 505     KR-A- 20130 136 332
US-A1- 2003 147 930

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **GUENICHE AUDREY ET AL: "Advances in Microbiome-Derived Solutions and Methodologies Are Founding a New Era in Skin Health and Care", PATHOGENS, vol. 11, no. 2, 20 January 2022 (2022-01-20), pages 121, XP055943801, DOI: 10.3390/pathogens11020121**
- **DATABASE GNPD [online] MINTEL; 4 October 2019 (2019-10-04), ANONYMOUS: "24H Deodorant Cream", XP093233014, retrieved from https://www.gnpd.com/sinatra/recordpage/ 6912857/ Database accession no. 6912857**

(52) Gemeinsame Patentklassifikation (CPC): (Forts.)

C-Sets
**A61K 31/19, A61K 2300/00;**
**A61K 31/192, A61K 2300/00**

**Beschreibung**

Die Erfindung betrifft kosmetische und dermatologische Zubereitungen, die eine Kombination aus Lävulinsäure und Anissäure umfassen. Die erfindungsgemäßen Zubereitungen eignen sich insbesondere zur Anwendung auf der Haut. Die Zubereitungen zeigen eine selektive antibakterielle Wirkung auf der Haut, indem sie gezielt anaerobe Bakterien der Spezies *Cutibacterium acnes* der Hautflora bekämpfen, während sie nur geringe oder keine bakterizide Wirkung gegen aerobe Bakterien der Spezies *S. epidermidis* aufweisen. Die erfindungsgemäßen Zusammensetzungen eignen sich demgemäß zur kosmetischen Hautpflege sowie auch zur dermatologischen Behandlung bestimmter Hauterkrankungen, wie z.B. Akne.

**HINTERGRUND DER ERFINDUNG**

Das Mikrobiom der menschlichen Haut ist eine komplexe Mischung verschiedener Gruppen von Mikroorganismen: anaerobe Bakterien, wie *Cutibacterium acnes* (*C. acnes*), besiedeln die Haut in unmittelbarer Nachbarschaft zu aeroben Bakterien, wie *Staphylococcus epidermidis (S. epidermidis)* und *Staphylococcus hominis* (*S. hominis*) sowie Pilzen.

Es ist bekannt, dass die Wechselwirkungen zwischen *S. epidermidis* und *C. acnes* für die Hautgesundheit von besonderer Bedeutung ist. *S. epidermidis* ist ein aerob wachsendes, kugelförmiges, grampositives Bakterium, das Katalase-positiv und Koagulase-negativ ist. Bei *C. acnes* handelt es sich um ein anaerob wachsendes, grampositives Bakterium. Es wird vermutet, dass beide Mikroorganismen auf gesunder Haut in einem verhältnismäßig stabilen Gleichgewicht vorkommen und eine Störung dieses Gleichgewichts, eine sogenannte Dysbiose, sich negativ auf den Zustand der Haut auswirken kann, indem Hautunreinheiten oder sogar Hauterkrankungen ausgelöst oder deren Entwicklung zumindest begünstigt werden. Es ist daher für die Behandlung oder Vorbeugung gegen solche Hautunreinheiten oder Hauterkrankungen von besonderer Bedeutung, Mittel bereitzustellen, mithilfe derer das Verhältnis von anaeroben zu anaeroben Mikroorganismen der Hautflora, und insbesondere das Verhältnis von *S. epidermidis* und *C. acnes,* gezielt verändert werden können. Da insbesondere das verstärkte Wachstum von anaeroben Bakterien, wie beispielsweise *C. acnes,* mit der Entwicklung von Hauterkrankungen einhergehen kann, werden insbesondere Zubereitungen benötigt, die selektiv gegen anaerobe Bakterien wirksam sind.

Die Anissäure ist eine methoxylierte Benzoesäure (4-Methoxybenzoesäure), die bakteriostatisch wirkt und der darüber hinaus eine anti-inflammatorische sowie auch fungizide Wirkung beigemessen wird. Anissäure wird u.a. in kosmetischen Produkten als Konservierungsmittel verwendet, um die Kontamination der Produkte durch Bakterien oder Pilze zu vermeiden. Zu diesem Zweck wird Anissäure diesen Produkten in einer Menge von 0,05-0,3 % zugesetzt.

Die Lävulinsäure, auch 4-Oxopentansäure oder 4-Oxovaleriansäure genannt, ist eine chemische Verbindung, die zu den $\gamma$-Ketosäuren gehört. International deklariert als "Levulinic Acid" ist die Säure und ihre Salze auf dem Gebiet der Hautpflege zum einen als Duftstoffzusatz gefragt, da ihr typischer Geruch angenehm an Karamell oder Vanille erinnert. Bekannt ist darüber hinaus eine antibakterielle und fungizide Wirkung von Lävulinsäure. Dies macht diesen Stoff auch als Hautpflegemittel bei unreiner Haut interessant. Da Lävulinsäure nicht als Konservierungsmittel deklariert ist, kann sie ideal zur Konservierung von natürlichen Kosmetika verwendet werden. Eine Verwendung der Lävulinsäure als Konservierungsmittel in kosmetischen Produkten ist hinreichend beschrieben worden, wobei die Substanz den zu konservierenden Produkten in einer Menge von 0,3-1,0% zugesetzt wird.

Konservierungsmittel in Kosmetika werden regelmäßig eingesetzt, um das Produkt vor Kontaminationen mit Bakterien und Pilzen zu schützen. Die mikrobiologische Stabilität von Kosmetika ist ein entscheidender Faktor für die Haltbarkeit und den gesundheitlichen Schutz der Verbraucher. In der europäischen Kosmetikverordnung sind Konservierungsstoffe als Stoffe definiert, die ausschließlich oder überwiegend dafür eingesetzt werden, um die Entwicklung von Mikroorganismen in kosmetischen Mitteln zu hemmen. Die hierfür zugelassenen Stoffe sind in der entsprechenden Positivliste in Anhang V der EU KVO aufgeführt.

Die Verwendung von Anissäure und Lävulinsäure als Konservierungsmittel ist im Stand der Technik beschrieben worden. EP 1 541 124 A2 beschreibt eine synergistische Wirkung zwischen zwei zur Konservierung verwendeten Wirkstoffen, nämlich Anissäure und C6-14-Fett-säuremonoglyceride. Die Mischung soll eine antimikrobielle Aktivität aufweisen, die sich zur Konservierung von kosmetischen Formulierungen nutzen lässt. In den Formulierungen entsteht somit ein breites antimikrobielles Mittel bei gleichzeitig guter Hautverträglichkeit. In der EP 2 735 301 A1 wird die Kombination von Lävulinsäure, dem Natriumsalz der Lävulinsäure (Natriumlävulinat) und Anissäure zur Konservierung eines Kosmetikprodukts offenbart.

US 2003/147930 A1 beschreibt kosmetische Lävulinsäure-haltige Zubereitungen, die bei der Bereinigung von Hautproblemen eingesetzt werden.

KR 2013 0136332 A offenbart ein Konservierungsmittel, das Lävulinsäure und Anissäure enthält und in Kosmetika Verwendung findet.

CN 109 157 505 A beschreibt ein Gel mit antibakterieller Wirkung, das eine Kombination aus Anis- und Lävulinsäure enthält, und topisch auf die Vaginalschleimhaut aufgetragen wird.

Während Anissäure und Lävulinsäure sich bei der Konservierung von Kosmetikprodukten umfänglich bewiesen haben, ist jedoch eine Verwendung dieser Substanzen zur gezielten Veränderung der Zusammensetzung der Hautflora bislang nicht bekannt. Wie oben erläutert besteht ein Bedürfnis nach kosmetischen und dermatologischen Zusammensetzungen, die zur Verbesserung des allgemeinen Zustands und der Gesundheit der Haut beitragen, indem sie gezielt in die Zusammensetzung der Hautflora eingreifen. Gleichzeitig sollen die Zusammensetzungen eine gute Hautverträglichkeit aufweisen.

## BESCHREIBUNG DER ERFINDUNG

Die vorliegende Erfindung basiert auf der überraschenden Erkenntnis, dass Anissäure in Kombination mit Lävulinsäure verwendet werden kann, um selektiv die Konzentration von anaeroben Bakterien in der Hautflora, insbesondere C. *acnes,* zu verringern. Die Erfindung betrifft folglich die Verwendung einer Kombination aus Anissäure und Lävulinsäure zur Reduktion anaerober Bakterien der Spezies *Cutibacterium acnes* und gleichzeitiger Bewahrung aerober Bakterien der Spezies *S. epidermidis* auf der Haut. Die Kombination erlaubt so nach der Applikation auf die Haut die Entfaltung einer selektiven antibakteriellen Wirkung auf der Haut.

Vorliegend offenbart wird eine Zusammensetzung für die gezielte Veränderung der Hautflora, wobei die Zusammensetzung folgendes umfasst:

(i) Anissäure oder ein antimikrobiell wirksames Salz davon, und
(ii) Lävulinsäure oder ein antimikrobiell wirksames Salz davon.

Die Zusammensetzungen enthalten vorzugsweise Anissäure. Es können jedoch auch Salze der Anissäure verwendet werden, sofern diese eine ausreichende antimikrobielle Wirksamkeit aufweisen. Zur Verwendung im Rahmen der vorliegenden Erfindung geeignete Salze der Anissäure weisen vorzugsweise eine antimikrobielle Aktivität auf, die zu mindestens 50% der Aktivität der Anissäure in einem entsprechenden in vitro Assay entspricht. Dies bedeutet, dass bei Verwendung gleicher Mengen der Anissäure und des Salzes der Anissäure zur Bestimmung der antimikrobiellen Aktivität in einem in vitro Assay das Salz der Anissäure im Vergleich zur Anissäure mindestens die Hälfte der antimikrobiellen Aktivität aufweisen muss. Geeignete Salze für die Verwendung im Rahmen der vorliegenden Erfindung sind Natrium- und Kalium-Anisat.

Zur Verwendung im Rahmen der vorliegenden Erfindung geeignete Salze der Lävulinsäure weisen vorzugsweise eine antimikrobielle Aktivität auf, die zu mindestens 50% der Aktivität der Lävulinsäure in einem entsprechenden in vitro Assay entspricht. Dies bedeutet, dass bei Verwendung gleicher Mengen der Lävulinsäure und des Salzes der Lävulinsäure zur Bestimmung der antimikrobiellen Aktivität in einem in vitro Assay das Salz der Lävulinsäure im Vergleich zur Lävulinsäure mindestens die Hälfte der antimikrobiellen Aktivität aufweisen muss. Geeignete Salze für die Verwendung im Rahmen der vorliegenden Erfindung sind Natrium- und Kalium-Lävulinat.

Insbesondere wird eine Zusammensetzung für die gezielte Veränderung der Hautflora offenbart, wobei die Zusammensetzung

(i) mehr als 0,3% (Gew./Gew.) Anissäure oder ein antimikrobiell wirksames Salz davon, und
(ii) mehr als 1,0% (Gew./Gew.) Lävulinsäure oder ein antimikrobiell wirksames Salz davon.

In einer besonders bevorzugten Ausführungsform umfasst die Zusammensetzung mindestens 0,5% (Gew./Gew.) Anissäure oder ein antimikrobiell wirksames Salz davon, und mindestens 1,5% (Gew./Gew.) Lävulinsäure oder ein antimikrobiell wirksames Salz davon. So beträgt die Menge der Anissäure oder des Anissäuresalzes in der Zusammensetzung vorzugsweise mindestens 0,75%, mindestens 1,0%, mindestens 1,5%, mindestens 2,0%, mindestens 2,5%, mindestens 3,0%, mindestens 3,5%, mindestens 4,5% oder mindestens 5,0% (Gew./Gew.). Die Menge der Lävulinsäure oder des Lävulinsäuresalzes in der Zusammensetzung beträgt vorzugsweise mindestens 2,0%, mindestens 2,5%, mindestens 3,0%, mindestens 3,5%, mindestens 4,5% oder mindestens 5,0% (Gew./Gew.).

Anders ausgedrückt umfasst die vorliegend offenbarte Zusammensetzung vorzugsweise zwischen 0,5 und 5,0% (Gew./Gew.) Anissäure oder ein antimikrobiell wirksames Salz davon, und zwischen 1,5 und 5,0% (Gew./Gew.) Lävulinsäure oder ein antimikrobiell wirksames Salz davon. Besonders bevorzugt ist eine Zusammensetzung, die zwischen 1,0 und 4,0% (Gew./Gew.) Anissäure oder Anissäuresalz und zwischen 2,0 und 4,0% (Gew./Gew.) Lävulinsäure oder Lävulinsäuresalz umfasst. Noch stärker bevorzugt ist eine Zusammensetzung, die zwischen 1,5 und 3,0% (Gew./Gew.) Anissäure oder Anissäuresalz und zwischen 2,0 und 3,0% (Gew./Gew.) Lävulinsäure oder Lävulinsäuresalz umfasst. Noch stärker bevorzugt ist eine Zusammensetzung, die zwischen 2,0 und 3,0% (Gew./Gew.) Anissäure oder Anissäuresalz und zwischen 2,5 und 3,0% (Gew./Gew.) Lävulinsäure oder Lävulinsäuresalz umfasst.

Die vorliegend offenbarten Zusammensetzungen können als einheitliche Formulierung vorliegen oder aber als Komponenten, die unmittelbar vor der Anwendung auf der Haut miteinander kombiniert werden. Im letztgenannten Fall sind die obigen Mengenangaben auf die Zusammensetzung anzuwenden, die sich aus der Kombination der Komponenten unmittelbar vor der Applikation ergibt. Ebenfalls offenbart werden Zusammensetzungen bei den die beiden antimikrobiell wirksamen Komponenten, nämlich die Anissäure oder das Salz davon und die Lävulinsäure oder das Salz davon als getrennte Formulierungen vorliegen, die nacheinander auf die Haut aufgetragen werden. So können die beiden antimikrobiell wirksamen Komponenten zeitlich versetzt im Abstand von etwa 5 Minuten, 10 Minuten, 15 Minuten, 20 Minuten, 24 Minuten oder 30 Minuten auf die Haut aufgetragen werden.

Die vorliegend offenbarten Zusammensetzungen können in verschiedenen Formen vorliegen. So sind z.B. eine Lösung, eine Suspension, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W) oder Öl-in-Wasser-in-Öl (O/W/O), eine Hydrodispersion oder Lipodispersion, oder auch ein Aerosol bevorzugte Zubereitungsformen. Vorzugsweise liegt die vorliegend offenbarte Zusammensetzung als Emulsionen vor, die neben den antimikrobiell wirksamen Komponenten weitere Substanzen enthält, wie z.B. Fette, Öle, Wachse und/oder andere Fettkörper, sowie Wasser und einen oder mehrere Emulgatoren, wie sie üblicherweise für einen solchen Typ der Formulierung verwendet werden. Solche Emulsionen können in vorteilhafter Weise als Creme oder Lotion formuliert sein. Es ist darüber hinaus auch möglich und vorteilhaft, die Kombinationen aus Anissäure und Lävulinsäure in wässrige Systeme bzw. Tensidzubereitungen zur Reinigung der Haut und/oder der Haare einzubringen.

Es ist dem Fachmanne natürlich bekannt, dass kosmetische und therapeutische Zusammensetzungen zumeist nicht ohne die üblichen Hilfs- und Zusatzstoffe denkbar sind. Dazu zählen beispielsweise Konsistenzgeber, Füllstoffe, Duftstoffe, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, sowie auch antimikrobiell, proteolytisch oder keratolytisch wirksame Substanzen usw., sofern deren Einsatz die antimikrobielle Aktivität der vorliegend offenbarten Kombination nicht signifikant inhibiert oder anderweitig zu Wider läuft.

Die vorliegend offenbarten Kombinationen aus Anissäure und Lävulinsäure können in vorteilhafter Weise in übliche kosmetische und dermatologische Zubereitungen eingearbeitet werden, welche wiederum in verschiedenen Formen vorliegen können. Die Zusammensetzungen können beispielsweise als Creme, Lotion, Gel, Salbe, Paste oder fester Stift vorliegen.

Die oben beschriebenen Zusammensetzungen eignen sich in besonderer Weise für die nicht-therapeutische, kosmetische und/oder für die therapeutische Behandlung der Haut, insbesondere der Gesichtshaut eines Menschen. In einer besonderen Ausführungsform betrifft die Erfindung eine wie oben beschriebene Zusammensetzung zur Verwendung in einem Verfahren zur Behandlung einer Hauterkrankung. Bei der Hauterkrankung handelt es sich vorzugsweise um eine Dermatitis. Die Behandlung von Akne *(Acne vulgaris)* mit den oben beschriebenen Zusammensetzungen ist besonders bevorzugt. Die therapeutische Wirkung der Zusammensetzungen ergibt sich in vorteilhafter Weise aus der gezielten Veränderung der Konzentration von anaeroben Bakterien der Spezies *Cutibacterium acnes* auf der Haut. Die Konzentration dieser anaeroben Bakterien wird verringert, wobei die Konzentration von anaeroben Bakterien der Spezies S. *epidermidis* im gleichen Habitat nicht oder nicht wesentlich verringert wird. Dadurch ergibt sich eine Verschiebung des Verhältnisses von *C. acnes* zu *S. epidermidis.*

Die vorliegend offenbarten Zusammensetzungen eignen sich ferner auch für eine nicht-therapeutische, kosmetische Verwendung. Eine solche nicht-therapeutische, kosmetische Verwendung kann der selektiven Verringerung der Anzahl der anaeroben Bakterien auf der Haut dienen, und insbesondere der selektiven Verringerung der Anzahl *C. acnes.* Auf diese Wiese können Hautunreinheiten beseitigt oder vermieden werden. Insbesondere können Verstopfungen von Talgdrüsen, wie sie z.B. bei Komedonen vorkommen, vermieden werden. Die nicht-therapeutische, kosmetische Verwendung kann damit das optische Erscheinungsbild der Haut erheblich verbessern. In einer anderen Ausführungsform betrifft die Erfindung ferner auch die nicht-therapeutische Verwendung einer wie oben definierten erfindungsgemäßen Zusammensetzung der Zubereitung zur Hautpflegehaut bei Akne.

Die Zusammensetzungen können im Rahmen der therapeutischen oder der nicht-therapeutischen, kosmetischen Behandlung ein- oder mehrfach auf die Haut aufgetragen werden. In einer Ausführungsform sind die vorliegend offenbarten Zusammensetzungen für die tägliche Applikation auf die Haut formuliert. In einer alternativen Ausführungsform sind die vorliegend offenbarten Zusammensetzungen für die einmalige Applikation auf die Haut formuliert. Überraschend wirkt die Kombination aus Anissäure und Lävulinsäure in den Zusammensetzungen selektiv, d.h. die anaeroben Bakterien der Spezies *C. acnes* werden auf der Haut reduziert und die aeroben Bakterien der Spezies *S. epidermidis* werden nicht oder nicht wesentlich angegriffen.

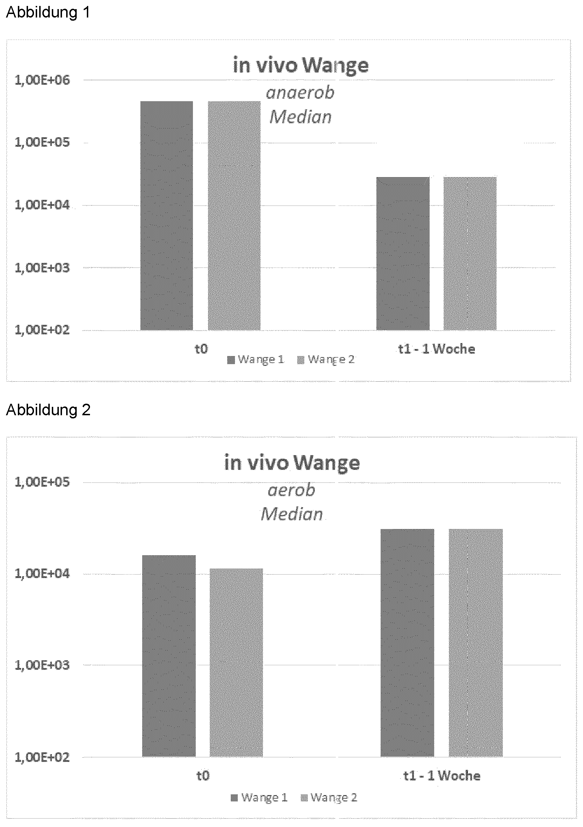

Der Vorteil der vorliegend offenbarten Zusammensetzungen liegt in der hohen antimikrobiellen Selektivität. Die Zusammensetzungen führen gezielt zu einem signifikanten Rückgang der Zellzahl von anaeroben Bakterien der Spezies *C. acnes,* welche die Haut besiedeln. Gleichzeitig wirken die Zusammensetzungen nicht oder nur geringfügig hemmend auf aeroben Bakterien der Spezies *S. epidermidis,* deren Anzahl auf der Haut nicht in signifikantem Umfang verringert wird. Wie in den nachstehend beschriebenen Beispielen erläutert und in den Abbildungen 1-2 gezeigt wird, führt die Verwendung der vorliegend offenbarten Zusammensetzungen zu einem Rückgang von 90% der anaeroben Bakterien, während die aeroben Bakterien sich im untersuchten Zeitraum sogar weiter vermehren.

Es wird vorliegend bevorzugt, dass die Anzahl von anaeroben Bakterien in einem bestimmten Hautareal nach Auftragen der Zusammensetzung um mindestens 50%, mindestens 60%, mindestens 70%, mindestens 80%, mindestens 90%, oder mindestens 95% im Vergleich zur Zellzahl im betreffenden Hautareal vor Auftragen der Zusammensetzung zurückgeht. Es ist darüber hinaus bevorzugt das die Anzahl von aeroben Bakterien in diesem Hautareal gleichzeitig um höchstens 25%, höchstens 20%, höchstens 15%, höchstens 10%, höchstens 5%, oder weniger im Vergleich zur Zellzahl im betreffenden Hautareal vor Auftragen der Zusammensetzung zurückgeht.

Es wird vorliegend besonders bevorzugt, dass die Anzahl von C. *acnes* in einem bestimmten Hautareal nach Auftragen der Zusammensetzung um mindestens 50%, mindestens 60%, mindestens 70%, mindestens 80%, mindestens 90%, oder mindestens 95% im Vergleich zur Zellzahl im betreffenden Hautareal vor Auftragen der Zusammensetzung zurückgeht. Es ist darüber hinaus bevorzugt das die Anzahl von *S. epidermidis* in diesem Hautareal gleichzeitig um höchstens 25%, höchstens 20%, höchstens 15%, höchstens 10%, höchstens 5%, oder weniger im Vergleich zur Zellzahl im betreffenden Hautareal vor Auftragen der Zusammensetzung zurückgeht.

Die Wirksamkeit der Zusammensetzungen wurde in verschiedenen Untersuchungen gezeigt, die in den nachfolgenden Beispielen beschrieben werden. In einem Test wurden dazu Bakterien von der Wange mittels Abstrichtechnik gewonnen und aerob und anaerob (d.h. bei reduziertem Sauerstoffgehalt) inkubiert. Nach 14 Tagen Anwendung wurden erneut Bakterien von den Probanden entnommen. Nach der Auswertung aller Probandendaten zeigte sich ein überaschendes Bild. Bei den aerob kultivierten Bakterien zeigten sich keine Auffäligkeiten, während es bei den anaerob kultivierten Bakterien schon nach einer Woche zu einer eine signifikanten Reduktion von mehr als 90% der Bakterienzellzahl kam. In weiteren in vitro Untersuchungen konnte dieses selektive Verhalten bestätigt werden.

**BEISPIELE**

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der jeweiligen Zubereitungen und Zusammensetzungen bezogen.

Beispiel 1: Herstellung von Bakteriensuspensionen und Wachstumsversuche

*S. epidermidis* (DSM 1798) wurde aus einem Kryoröhrchen (EN 12353) entnommen, auf einer CASO-Agarplatte ausgestrichen und bei 37°C 24 h inkubiert. Anschließend wurde eine zweite Passage angelegt. Mit der zweiten Passage wurden 10 ml BHI-Medium und 5 g Glasperlen (4 mm Durchmesser) angeimpft und 3 min geschüttelt. Danach wurde dieser Ansatz auf eine OD600 von 0,1 eingestellt und anschließend 1:10 mit Verdünnungsmittel verdünnt. Diese Bakteriensuspension wurde anschließend für den Growth-Assay eingesetzt.

Test - Wachstumsversuch mit *S. epidermidis*

In jedes Well einer 24-Well-Platte wurden 10µL Bakteriensuspension gegeben und komplett trocknen gelassen (mind. 45 min unter der Sterilwerkbank). Nach der Trocknung wurden 10mg der zu untersuchenden Formulierung (siehe Tabelle 1) aufpipettiert und mittels T-Spatel im Well verteilt. Die Kontrolle blieb unbehandelt. Die Platte wurde für die Inkubationsdauer (6h / 24h) im Feuchtbrutschrank bei 37°C und 90% Luftfeuchtigkeit ohne Deckel gelagert. Nach Ablauf der Inkubationszeit wurde in jedes Well 500µL Abspülpuffer gegeben und 1min im Ultraschallbad abgespült. 100µL des erhaltenen Überstandes wurden zu 900µL Neutralisationsmittel gegeben und anschließend auf CASO-Agar mittels Spiralplater ausplattiert. Die Agar-platten wurden bei 37°C für 24h inkubiert, und die Kolonien wurden anschließend mit dem I+L Countermat ausgezählt.

*C. acnes* (DSM 1897) wurde aus einem Kryoröhrchen (EN 12353) auf einer Agarplatte (COST-Agar) ausgestrichen und bei 37°C 5 Tage in der anaeroben Box inkubiert. Mit dieser Kultur wurden 10 ml anaeroben Medium im CELLSTAR CELLreactor - Röhrchen angeimpft und 18h in der anaeroben Box inkubiert. Danach wurde dieser Ansatz auf eine OD600 von 0,5 eingestellt und anschließend 1:10 mit anaerobem Medium verdünnt. Diese Bakteriensuspension wurde anschließend für den Growth-Assay eingesetzt.

Test - Wachstumsversuch mit C. *acnes*

In jedes Well einer 24-Well-Platte wurden 10µL Bakteriensuspension gegeben und komplett trocknen gelassen (mind. 45min unter der Sterilwerkbank). Nach der Trocknung wurden 10mg der zu untersuchenden Formulierung (siehe Tabelle 1) aufpipettiert und mittels T-Spatel im Well verteilt. Die Kontrolle blieb unbehandelt. Die Platte wurde für die Inkubationsdauer (6h / 24h) ohne Deckel in einer anaeroben Box (mit Zellstoff ausgelegt + 50mL Wasser) im Brutschrank bei 37°C gelagert. Nach Ablauf der Inkubationszeit wurde in jedes Well 500µL Abspülpuffer gegeben und 1min im Ultraschallbad abgespült. 100µL des erhaltenen Überstandes wurden zu 900µL Neutralisationsmittel gegeben und anschließend auf COST-Agar mittels Spiralplater ausplattiert. Die Agarplatten wurden bei 37°C für 5 Tage inkubiert, und die Kolonien wurden anschließend mit dem I+L Countermat ausgezählt.

Im Rahmen der Auswertung wurde der Reduktionsfaktor bestimmt. Der Reduktionsfaktor ist der Faktor, um den die Zellzahl (CFU) der unterschiedlichen Zeitpunkte im Vergleich zur T0-Kontrolle oder untereinander reduziert wird.

$$\frac{CFU(Kontrolle)}{CFU\ (Testsubstanz)} = Reduktionsfaktor$$

Das Studiendesign ist so ausgelegt, dass eine antibakterielle Wirksamkeit der Testformulierungen durch Vergleich zur unbehandelten Kontrolle nachgewiesen wird.

Tabelle 1: getestete Formulierungen und Ergebnisse der Wachstumsversuche

| Formulierung Nr. | 1 | 2 | 3 | 4 | 5 | 6 |
|---|---|---|---|---|---|---|
| INCI | | | | | | |
| | % | % | % | % | % | % |
| p-Anisic Acid | 0,1 | 0,1 | 0,1 | 0,1 | 0,1 | 0,1 |
| Levulinic Acid (Aqua/Glycerin/ Sodium Levulinate) | 0,5 | 0,5 | 0,5 | 0,5 | 0,5 | 0,5 |
| Glycerin + Arctium Lappa Fruit Extract | 1,2 | 1,2 | 1,2 | 0 | 0 | 0 |
| Ethylhexylglycerin | 0,1 | 0,1 | 0 | 0,1 | 0,2 | 0,1 |
| Decylene Glycol | 0 | 0,1 | 0,1 | 0,1 | 0 | 0 |
| Sodium Hyaluronate + Aqua | 0 | 0 | 0,1 | 0 | 0 | 0 |
| Maltodextrin + Chamomilla Recutita Flower Extract | 0 | 0 | 0 | 0,6 | 0 | 0 |
| Dicaprylyl Ether | 2,8 | 3,5 | 0 | 3,5 | 2,8 | 2,8 |
| Simmondsia Chinensis Seed Oil | 3,5 | 3,5 | 0 | 3,5 | 3,5 | 3,5 |
| Butyrospermum Parkii Butter | 2 | 2 | 0 | 2 | 3,5 | 2 |
| Prunus Amygdalus Dulcis Oil | 3 | 3,5 | 0 | 3,5 | 5 | 3 |
| Coco-Caprylate/Caprate | 1,2 | 0 | 0 | 0 | 0 | 1,2 |
| Argania Spinosa Kernel Oil | 0,1 | 0,1 | 0,1 | 0 | 0,1 | 0 |
| Caprylic/Capric Triglyceride | 0 | 1 | 0 | 1 | 0 | 0 |
| Brassica Campestris Seed Oil | 0 | 0 | 6 | 0 | 0 | 0 |
| Glyceryl Stearate | 2 | 2 | 0 | 2 | 2 | 2 |
| Glyceryl Caprylate | 0,3 | 0,3 | 0,3 | 0,3 | 0,3 | 0,3 |
| Sodium Stearoyl Glutamate | 0,3 | 0,3 | 0 | 0,3 | 0,3 | 0,3 |
| Sodium Cetearyl Sulfate | 0 | 0 | 0,2 | 0 | 0 | 0 |
| Parfum | 0,35 | 0,35 | 0,35 | 0 | 0,3 | 0,3 |
| Glycerin | 8,4 | 8,4 | 9 | 8,4 | 8,4 | 8,4 |
| Cetearyl Alcohol | 5 | 5 | 0 | 5 | 5 | 5 |
| Xanthan Gum | 0,3 | 0,3 | 0,2 | 0,3 | 0,3 | 0,3 |
| Cetyl Alcohol | 0 | 0 | 3 | 0 | 0 | 0 |
| Hydroxypropyl Starch Phosphate + Aqua | 0 | 0 | 3 | 0 | 0 | 0 |
| Alcohol Denat. + Aqua | 4 | 4 | 4 | 4 | 4 | 4 |
| Aqua | ad 100 | ad 100 | ad 100 | ad 100 | ad 100 | ad 100 |
| Reduktionsfaktoren | 1 | 2 | 3 | 4 | 5 | 6 |

(fortgesetzt)

| Formulierung Nr. | 1 | 2 | 3 | 4 | 5 | 6 |
|---|---|---|---|---|---|---|
| INCI | | | | | | |
| | % | % | % | % | % | % |
| Staphylococcus epidermidis nach 6h | 1 | 1 | 10 | 1 | 1 | 1 |
| Cutibacterium acnes nach 6h | 40 | 134 | 1651 | 214 | 813 | 71 |

Die Auszählung zeigte, dass die Anzahl der aeroben *S. epidermidis* Bakterien durch die Formulierung, welche Anissäure und Lävulinsäure enthielten, nicht oder nicht wesentlich beeinträchtigt wurde. In den Ansätzen von S. *epidermidis* wurden nur geringe Reduktionsfaktoren gemessen. Hingegen wurde die Anzahl der anaeroben C. *acnes* Bakterien durch die Formulierungen signifikant reduziert.

Beispiel 2: ATA-Test

Der ATA Test dient der Bestimmung der antibakteriellen Wirksamkeit von kosmetischen Formulierungen nach 2x täglicher Anwendung. Die Testdauer beträgt i.d.R. 1 Woche. Die Anwendung der Testformulierung und die Messung der Wirksamkeit erfolgen in dem verbraucherrelevanten Areal, der Wange. Zielgröße ist die Keimzahl der aeroben und anaeroben Bakterien.

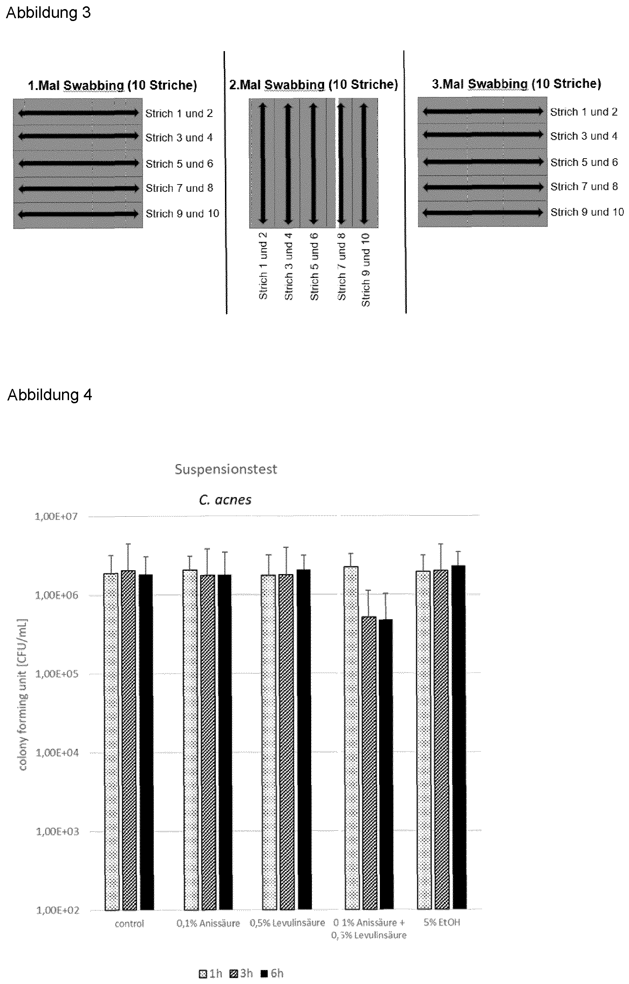

Alle Probanden haben eine Vorkonditionierung von 3 Tagen mit einem vorgegebenen Waschgel durchlaufen. Nach Abspülen der Wangen wird die in Tabelle 1 beschriebene "Testformulierung 5" nach Vorschrift aufgetragen. Anschließend erfolgt ein Abstrich mittels eines Floq Swabs. Es werden 3 x 10 Striche auf einer 5 cm x 5 cm großen Fläche der Wange in einem definierten Muster abgestrichen. Das Muster des Abstrichs ist in Abbildung 3 dargestellt. Während der Probenabnahmezyklen wird der Floq-Swab in einem Abspülpuffer getränkt, und nach der dritten Probennahme verbleibt der Swab im Abspülpuffer. Die Lösung wird direkt verdünnt und jede Probe wird mittels Spiralplater auf 2 Agar-Platten ausplattiert. Der Abstrich wird dann zu den entsprechend vorgegebenen Zeitpunkten, meistens nach 1 Woche, wiederholt. Zum Schluss wurde die eine Hälfte der Agar-Platten bei 37°C 5 Tage unter Ausschluss von Sauerstoff bebrütet und die andere Hälfte der Agar-Platten bei 37°C über 24h bebrütet. Anschließend wurden die Agar-Platten mit Hilfe des Countermats ausgewertet.

Die Ergebnisse des ATA-Tests sind in den Abbildungen 1 und 2 gezeigt. Abbildung 1 zeigt die Ergebnisse der anaeroben Kultivierung. Es ist zu erkennen, dass die Zellzahl der anaeroben Bakterien durch Behandlung mit der Testformulierung, die eine Kombination aus Anissäure und Lävulinsäure enthält, deutlich zurückgegangen ist. Eine solche Verringerung der Zellzahl ist in Abbildung 2, welche die Ergebnisse der aeroben Kultivierung zeigt, nicht zu erkennen. Vielmehr konnte hier sogar eine Zunahme der Zellzahl verzeichnet werden. Die Abbildungen 1 und 2 zeigen somit eindrucksvoll die selektive Wirksamkeit der vorliegend offenbarten Kombination aus Anissäure und Lävulinsäure.

Beispiel 3: Suspensions-Tests

Die Wirkung der vorliegend offenbarten Formulierungen wurde in Suspensionstests mit *S. epidermidis* und *C. acnes* weiter untersucht. Glycerin-Stammkulturen von *S. epidermidis* (DSM 1798) und *C. acnes* (DSM 1897), die nach der europäischen Norm 12353 angelegt worden waren, wurden im Kühlschrank bei 5°C gelagert. Aus diesen flüssigen Stammkulturen wurde eine erste Passage hergestellt. Hierbei wurde mittels steriler Einmalimpföse die entsprechende Kultur auf einer Agarplatte (*C. acnes:* COST-Agar, *S. epidermidis:* COAST-Agar) ausgestrichen und anschließend bebrütet. Die Kulturen von C. *acnes* wurden für 5 Tage bei 37°C bebrütet, während die Kulturen von *S. epidermidis* für 24 h bei 37°C bebrütet wurden. Aus dieser ersten Passage wurde anschließend eine zweite Passage hergestellt, in dem man einige Kolonien mit einer sterilen Einmalimpföse aufnimmt und diese anschließend auf eine neue Agarplatte ausstreicht. Diese zweite Passage wurde anschließend für 5 Tage bzw. 24h bei der entsprechenden Temperatur bebrütet. Die erhaltene zweite Passage wurde anschließend für den Suspensionstest eingesetzt.

Zur Herstellung der Suspensionen wurden einige Kolonien der zweiten Passage mit einer sterilen Impföse abgenommen und zu 10mL Medium in ein Schikanekolben mit 5g Glasperlen (Ø 4mm) gegeben und 3min leicht geschüttelt. Anschließend wurde mittels optischer Dichte (OD) die ungefähre Zellzahl pro mL eingestellt. Die optische Dichte wurde mit dem Eppendorf Photometer bei 600 nm bestimmt. Die erhaltene OD wurde auf einen Wert um 0,8 (±0,01) eingestellt werden und anschließend nochmals 1:50 verdünnt. Mit dieser Verdünnungsstrategie erhielt man eine Zellzahl von etwa $1 \times 10^6$.

Jeweils 50$\mu$L der antimikrobiellen Rohstofflösung und 450$\mu$L Medium wurden für die Testformulierungen in sterilisierten Eppendorf Reaktionsgefäßen vorgelegt (*C. acnes:* Anaeroben-Medium, *S. epidermidis:* BHI-Medium). Für

die Positivkontrolle wurden 500μL Medium vorgelegt. Nach Zugabe von 500μL der hergestellten Kultursuspension wurden zu verschiedenen Zeitpunkten 1:10-Verdünnungen aller Proben hergestellt (nach 1h, 3h, und 6h). Für die Positivkontrolle wurde zusätzlich direkt nach der Kulturzugabe (0min) eine Verdünnung hergestellt und mittels Spiralplatter ausplattiert. Über die gesamte Testdauer wurden die Proben in einem Eppendorf-Schüttler bei 1000rpm und 30°C bzw. 37°C gelagert. Für die 1:10-Verdünnungen wurden 900μL Neutralisationsmedium in Eppendorf Reaktionsgefäßen vorgelegt und zu den Abnahmezeitpunkten wurden 100μL der Kultur-Rohstoff-Suspension dazu gegeben. Nachdem der Test abgeschlossen war, wurden alle Agarplatten zum Bebrüten in den Brutschrank eingelagert und nach der Inkubation am Countermat ausgewertet.

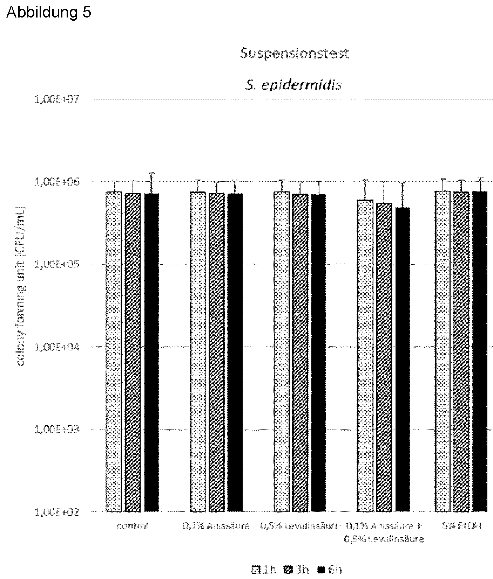

Die Ergebnisse werden in den Abbildungen 4 und 5 gezeigt. Es ist zu erkennen, dass die Verwendung von Anissäure, Lävulinsäure oder einer Kombination beiden Substanzen das Wachstum des aeroben Bakteriums *S. epidermidis* nicht signifikant beeinträchtigt. Bei dem anaeroben Bakterium *C. acnes* hingegen führt zwar die alleinige Verwendung von Anissäure oder Lävulinsäure nicht zu einer Verringerung der Zellzahl. Allerdings führt die kombinierte Verwendung zu einer signifikanten selektiven antimikrobiellen Wirkung.

## Patentansprüche

1. Nicht-therapeutische Verwendung einer Kombination aus (i) Anissäure oder einem antimikrobiell wirksamen Salz davon und (ii) Lävulinsäure oder einem antimikrobiell wirksamen Salz davon, zur selektiven Verringerung der Anzahl von anaeroben Bakterien der Spezies *Cutibacterium acnes* auf der Haut, wobei die Anzahl der aeroben Bakterien der Spezies *Staphylococcus epidermidis* auf der Haut nicht in signifikantem Umfang verringert wird.

2. Nicht-therapeutische Verwendung nach Anspruch 1, wobei es sich bei der Haut um die Gesichtshaut handelt.

3. Nicht-therapeutische Verwendung nach einem der Ansprüche 1-2, wobei die beiden Komponenten der Kombination gleichzeitig oder nacheinander auf die Haut aufgetragen werden.

4. Nicht-therapeutische Verwendung nach einem der Ansprüche 1-3, wobei die Kombination in Form einer einheitlichen Zusammensetzung verabreicht wird, die 0,01 bis 5,0% (Gew./Gew.), und vorzugsweise 0,1 bis 1,0% (Gew./Gew.), Anissäure oder ein antimikrobiell wirksames Salz davon umfasst.

5. Nicht-therapeutische Verwendung nach einem der Ansprüche 1-4, wobei die Kombination in Form einer einheitlichen Zusammensetzung verabreicht wird, die 0,01 bis 5,0% (Gew./Gew.), und vorzugsweise 0,1 bis 1,0% (Gew./Gew.), Lävulinsäure oder ein antimikrobiell wirksames Salz davon umfasst.

## Claims

1. Non-therapeutic use of a combination of (i) anisic acid or an antimicrobially active salt thereof and (ii) levulinic acid or an antimicrobially active salt thereof for selectively reducing the number of anaerobic bacteria of the species *Cutibacterium acnes* on the skin, wherein the number of aerobic bacteria of the species *Staphylococcus epidermidis* on the skin is not significantly reduced.

2. Non-therapeutic use according to claim 1, wherein the skin is facial skin.

3. Non-therapeutic use according to any of claims 1-2, wherein the two components of the combination are applied to the skin simultaneously or one after the other.

4. Non-therapeutic use according to any of claims 1-3, wherein the combination is administered in the form of a uniform composition comprising 0.01 to 5.0% (w/w), and preferably 0.1 to 1.0% (w/w), anisic acid or an antimicrobially active salt thereof.

5. Non-therapeutic use according to any one of claims 1-4, wherein the combination is administered in the form of a uniform composition comprising 0.01 to 5.0% (w/w), and preferably 0.1 to 1.0% (w/ w/w), levulinic acid or an antimicrobially effective salt thereof.

**Revendications**

1. Utilisation non thérapeutique d'une association (i) d'acide anisique ou d'un sel à activité antimicrobienne de celui-ci et (ii) d'acide lévulinique ou d'un sel à activité antimicrobienne de celui-ci, pour la réduction sélective du nombre de bactéries anaérobies de l'espèce *Cutibacterium acnes* sur la peau, le nombre des bactéries aérobies de l'espèce *Staphylococcus epidermidis* sur la peau n'étant pas réduit de manière significative.

2. Utilisation non thérapeutique selon la revendication 1, dans laquelle pour ce qui est de la peau il s'agit de la peau du visage.

3. Utilisation non thérapeutique selon l'une quelconque des revendications 1-2, dans laquelle les deux composants de l'association sont appliqués sur la peau simultanément ou l'un après l'autre.

4. Utilisation non thérapeutique selon l'une quelconque des revendications 1-3, dans laquelle l'association est administrée sous forme d'une composition homogène qui comprend 0,01 à 5,0 % (p/p) et de préférence 0,1 à 1,0 % (p/p) d'acide anisique ou d'un sel à activité antimicrobienne de celui-ci.

5. Utilisation non thérapeutique selon l'une quelconque des revendications 1-4, dans laquelle l'association est administrée sous forme d'une composition homogène qui comprend 0,01 à 5,0 % (p/p) et de préférence 0,1 à 1,0 % (p/p) d'acide lévulinique ou d'un sel à activité antimicrobienne de celui-ci.

Abbildung 1

Abbildung 2

Abbildung 3

| 1.Mal Swabbing (10 Striche) | 2.Mal Swabbing (10 Striche) | 3.Mal Swabbing (10 Striche) |
|---|---|---|

Strich 1 und 2
Strich 3 und 4
Strich 5 und 6
Strich 7 und 8
Strich 9 und 10

Strich 1 und 2
Strich 3 und 4
Strich 5 und 6
Strich 7 und 8
Strich 9 und 10

Strich 1 und 2
Strich 3 und 4
Strich 5 und 6
Strich 7 und 8
Strich 9 und 10

Abbildung 4

Suspensionstest

*C. acnes*

control   0,1% Anissäure   0,5% Levulinsäure   0 1% Anissäure + 0,5% Levulinsäure   5% EtOH

colony forming unit [CFU/mL]

1,00E+07
1,00E+06
1,00E+05
1,00E+04
1,00E+03
1,00E+02

⊠ 1h  ▨ 3h  ■ 6h

Abbildung 5

**Suspensionstest**

*S. epidermidis*

**IN DER BESCHREIBUNG AUFGEFÜHRTE DOKUMENTE**

**In der Beschreibung aufgeführte Patentdokumente**

- EP 1541124 A2
- EP 2735301 A1
- US 2003147930 A1
- KR 20130136332 A
- CN 109157505 A